# EUROPÄISCHE PATENTSCHRIFT

(11) **EP 1 417 264 B1**
(45) Veröffentlichungstag und Bekanntmachung des Hinweises auf die Patenterteilung: **15.10.2008**
(21) Anmeldenummer: 02764768.4
(22) Anmeldetag: 24.07.2002
(51) Int. Cl.: C09B 29/20, C07D 239/96

(54) **NEUE AZOPIGMENTE**
NOVEL AZO PIGMENTS
NOUVEAUX PIGMENTS AZOIQUES

(30) Priorität: 07.08.2001 DE 10138770
(43) Veröffentlichungstag der Anmeldung: 12.05.2004
(73) Patentinhaber: Clariant Produkte (Deutschland) GmbH, 65929 Frankfurt am Main (DE)
(72) Erfinder: GRIMM, Felix, W., 65719 Hofheim (DE); METZ, Hans, Joachim, 64285 Darmstadt (DE); WACKER, Andreas, 68165 Mannheim (DE)
(86) Internationale Anmeldenummer: PCT/EP2002/008227
(87) Internationale Veröffentlichungsnummer: WO 2003/014227

(56) Entgegenhaltungen:
- EP-A- 0 359 708
- DE-B- 1 289 928
- GB-A- 1 001 706

## Beschreibung

Die vorliegende Erfindung betrifft neue Azopigmente, die auf N-substituierten 6-(2'-Hydroxy-3'-naphthoylamino)chinazolin-2,4-dion-Kupplungskomponenten aufbauen.

Azopigmente der 2-Hydroxy-3-naphtoesäureanilide, wie z.B. C.I. Pigment Red 2, 9, 12, 14, 112, 144, 146, 147, 170, 175, 184, 185, 187, 188, 210, 247 oder 256 besitzen große Bedeutung zur Einfärbung von hochmolekularen organischen Materialien. Ein Nachteil dieser Pigmente ist ihre den heutigen Ansprüchen nicht mehr genügende Licht- und Wetterechtheit in anspruchsvollen Einsatzgebieten.

Ebenfalls bekannt sind Kupplungskomponenten, die durch Kondensation von 2-Hydroxy-3-naphtoesäure mit 6-Amino-chinazolin-2,4-dion erhalten werden (DE-A-12 89 928). Die damit hergestellten Farbmittel weisen aber trübe, wenig brillante Farbtöne und ungünstige rheologische Eigenschaften auf, lassen sich im Anwendungsmedium schlecht dispergieren und sind farbschwach.

Aufgabe der vorliegenden Erfindung war es, neue Pigmente mit hoher Licht- und Wetterechtheit sowie brillanten Farbtönen bereitzustellen, die sich außerdem gut im Anwendungsmedium verarbeiten lassen.

Es wurde nun gefunden, dass Verbindungen der nachstehend definierten allgemeinen Formel (I) überraschenderweise diese Anforderungen erfüllen.

Gegenstand der vorliegenden Erfindung sind Azopigmente der Formel (I) 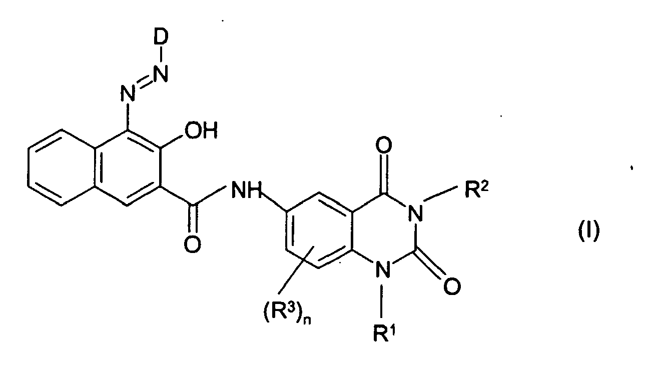 worin D den Rest einer Diazokomponente darstellt,
R¹ und R² gleich oder verschieden sind und Wasserstoff, einen gegebenenfalls durch Hydroxy, Amino, Halogen oder Methoxy substituierten C₁-C₄-Alkylrest, oder einen gegebenenfalls mit 1, 2 oder 3 Substituenten aus der Gruppe C₁-C₄-Alkyl, C₁-C₄-Alkoxy, F, Cl, Br, CF₃, NO₂, OH, CONH₂, CONH(C₁-C₄-alkyl), CON(C₁-C₄-alkyl)₂, COO(C₁-C₄-alkyl), SO₂NH₂, SO₂NH(C₁-C₄-alkyl) und SO₂N(C₁-C₄-alkyl)₂ substituierten (C₆-C₁₀)-Arylrest bedeuten,
mit der Maßgabe, dass R¹ und R² nicht gleichzeitig Wasserstoff sind;
R³ Wasserstoff, C₁-C₄-Alkyl, Halogen, insbesondere Fluor, Chlor, Brom, Trifluormethyl, C₁-C₄-Alkoxy, NO₂, oder CN bedeutet, und
n die Zahl 1, 2 oder 3 ist.

Die allgemeine Formel (I) ist als idealisierte Darstellung zu verstehen und umfasst auch die entsprechenden tautomeren Verbindungen, sowie die möglichen Konfigurationsisomere jeder tautomeren Form.

Bevorzugte Reste R¹ und R² sind Wasserstoff, Methyl, Ethyl oder Phenyl, mit der vorstehend genannten Maßgabe.
Besonders bevorzugt sind Azopigmente, worin R¹ Methyl oder Ethyl, und R² Wasserstoff bedeuten.

Bevorzugte Reste R³ sind Wasserstoff, Methyl, Ethyl, Methoxy, Ethoxy, F, Cl, CF₃ und Nitro.

Bevorzugte Reste D sind (C₆-C₁₂)-Arylreste, die mit 1, 2 oder 3 Substituenten aus der Gruppe C₁-C₄-Alkyl, C₁-C₄-Alkoxy, Phenyl, Chlorphenyl, Dichlorphenyl, F, Cl, Br, CF₃, NO₂, NH₂, COOH, OH, CONH₂, CONH(C₁-C₄-alkyl), CON(C₁-C₄-alkyl)₂, COO(C₁-C₄-alkyl), SO₂NH₂, SO₂NH(C₁-C₄-alkyl) und SO₂N(C₁-C₄-alkyl)₂ substituiert sind, oder ein (C₆-C₁₀)-Arylrest, an den ein fünf- oder sechsgliedriger heterocyclischer Ring mit 1, 2 oder 3 Heteroatomen aus der Gruppe N, O und S annelliert ist.

Gegenstand der vorliegenden Erfindung ist auch ein Verfahren zur Herstellung der Verbindungen der Formel (I), dadurch gekennzeichnet, dass man ein aromatisches Amin der Formel D-NH₂ diazotiert und mit einer oder mehreren Verbindungen der allgemeinen Formel (II), 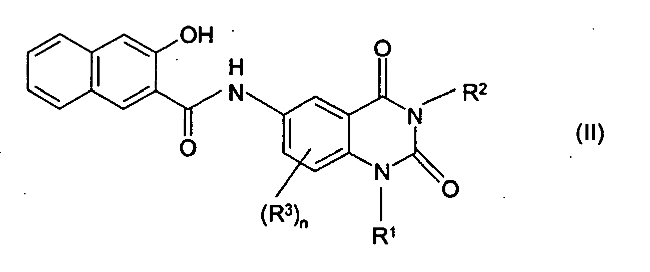 kuppelt.

Besonders bevorzugte aromatische Amine D-NH₂ sind:
2-Chloranilin, 4-Methyl-2-nitro-anilin, 4-Chlor-2-nitro-anilin, 3,3'-Dichlor-biphenyl-4,4'-diamin, 3,3'-Dimethyl-biphenyl-4,4'-diamin, 4-Methoxy-2-nitro-anilin, 2-Methoxy-4-nitro-anilin, 4-Nitroanilin, 5-Amino-isophthalsäuredimethylester, Anthranilsäure-methylester, 2-Trifluormethyl-anilin, 2-Aminoterephthalsäuredimethylester, 2-Methoxy-anilin, 2,4-Dinitro-anilin, 3-Amino-4-chlor-benzamid, 3-Amino-4-methyl-benzamid, 2,5-Dichlor-anilin, 2-Chlor-4-nitro-anilin, 2-Methyl-5-nitro-anilin, 2-Methyl-4-nitro-anilin, 2,4,5-Trichlor-anilin, 4-Aminobenzamid, 4-Amino-5-methoxy-N,N-dimethyl-benzolsulfonamid, 2-Amino-N-(2,5-dichloro-phenyl)-terephthal-säuremonomethylester, 2-Amino-benzoesäurebutylester, 2-Chloro-5-trifluoromethyl-anilin, 4-Amino-2,5-dichloro-N-methyl-benzolsulfonamid, 4-Amino-2,5-dichloro-N,N-dimethyl-benzolsulfonamid, 6-Amino-1 H-chinazolin-2,4-dion, 4-Amino-2,5-dimethoxy-N-methyl-benzolsulfonamid, 2,4-Dichloranilin, 5-Chlor-2-methylanilin, 2-Aminobenzoesäure, 3-Amino-4-methoxy-N-phenylbenzamid, 3-Amino-N-[4-(aminocarbonyl)phenyl]-4-methoxybenzamid, 4-Amino-5-methoxy-2-methyl-N-methylbenzolsulfonamid, 5-Amino-benzimidazol-2-on, 6-Amino-chinoxalin-2,3-dion, 6-Methoxy-7-aminochinoxalin-2,3-dion, 4-Amino-2,5-dimethoxy-N-phenylbenzol-sulfonamid, 4-Amino-N-[4-(aminocarbonyl)phenyl]-benzamid, 1,2-Bis-(2-amino-phenoxy)-ethan, 2-Ethoxyanilin und 4-Ethoxyanilin.

Die Temperaturen der Azokupplung betragen normalerweise von -10 bis +90°C, bevorzugt von -5 bis +80°C, insbesondere von 0 bis 70°C. Die Azokupplungsreaktion erfolgt vorzugsweise in wässriger Lösung oder Suspension, es können aber auch organische Lösemittel, gegebenenfalls im Gemisch mit Wasser eingesetzt werden, beispielsweise Alkohole mit 1 bis 10 C-Atomen, wie beispielsweise Methanol, Ethanol, n-Propanol, Isopropanol, Butanole, wie n-Butanol, sek.-Butanol, tert.-Butanol, Pentanole, wie n-Pentanol, 2-Methyl-2-butanol, Hexanole, wie 2-Methyl-2-pentanol, 3-Methyl-3-pentanol, 2-Methyl-2-hexanol, 3-Ethyl-3-pentanol, Octanole, wie 2,4,4-Trimethyl-2-pentanol, Cyclohexanol; oder Glykole, wie Ethylenglykol, Diethylenglykol, Propylenglykol, Dipropylenglykol, oder Glycerin; Polyglykole, wie Polyethylenglykole oder Polypropylenglykole; Ether, wie Methylisobutylether, Tetrahydrofuran oder Dimethoxyethan; Glykolether, wie Monomethyl- oder Monoethylether des Ethylen- oder Propylenglykols, Diethylenglykolmonomethyl-ether, Diethylenglykolmonoethylether, Butylglykole oder Methoxybutanol; Ketone, wie Aceton, Diethylketon, Methylisobutylketon, Methylethylketon oder Cyclohexanon; aliphatische Säureamide, wie Formamid, Dimethylformamid, N-Methylacetamid oder N,N-Dimethylacetamid; Harnstoffderivate, wie Tetramethylharnstoff; oder cyclische Carbonsäureamide, wie N-Methylpyrrolidon, Valero- oder Caprolactam; Ester, wie Carbonsäure-C₁-C₆-alkylester, wie Ameisensäurebutylester, Essigsäureethylester oder Propionsäurepropylester; oder Carbonsäure-C₁-C₆-glykolester; oder Glykoletheracetate, wie 1-Methoxy-2-propylacetat; oder Phthalsäure- oder Benzosäure-C₁-C₆-alkylester, wie Benzoesäureethylester; cyclische Ester, wie Caprolacton; Nitrile, wie Acetonitril oder Benzonitril; aliphatische oder aromatische Kohlenwasserstoffe, wie Cyclohexan oder Benzol; oder durch Alkyl, Alkoxy, Nitro oder Halogen substituiertes Benzol, wie Toluol, Xylole, Ethylbenzol, Anisol, Nitrobenzol, Chlorbenzol, o-Dichlorbenzol, 1,2,4-Trichlorbenzol oder Brombenzol; oder andere substituierte Aromaten, wie Benzoesäure oder Phenol; aromatische Heterocyclen, wie Pyridin, Morpholin, Picolin oder Chinolin; sowie Hexamethylphosphorsäuretriamid, 1,3-Dimetyl-2-imidazolidinon, Dimethylsulfoxid und Sulfolan. Die genannten Lösemittel können auch als Mischungen eingesetzt werden. Vorzugsweise werden mit Wasser mischbare Lösemittel eingesetzt.

Im erfindungsgemäßen Verfahren können auch übliche Hilfsmittel wie beispielsweise Kupplungshilfsmittel, Tenside, pigmentäre und nichtpigmentäre Dispergiermittel, Füllstoffe, Stellmittel, Harze, Wachse, Entschäumer, Antistaubmittel, Extender, Farbmittel zum Nuancieren, Konservierungsmittel, Trocknungsverzögerungsmittel, Additive zur Steuerung der Rheologie, Netzmittel, Antioxidantien, UV-Absorber, Lichtstabilisatoren, oder eine Kombination davon eingesetzt werden.
Die Gesamtmenge der zugegebenen Hilfsmittel kann 0 bis 40 Gew.-%, bevorzugt 1 bis 30 Gew.-%, besonders bevorzugt 2,5 bis 25 Gew.-%, bezogen auf das Azopigment, betragen.

Als Tenside kommen anionische oder anionaktive, kationische oder kationaktive und nichtionische Substanzen oder Mischungen dieser Mittel in Betracht.
Als anionaktive Substanzen kommen beispielsweise Fettsäuretauride, FettsäureN-methyltauride, Fettsäureisethionate, Alkylphenylsulfonate, Alkylnaphthalinsulfonate, Alkylphenolpolyglykolethersulfate, Fettalkoholpolyglykolethersulfate, Fettsäureamid-polyglykolethersulfate, Alkylsulfosuccinamate, Alkenylbernsteinsäurehalbester, Fettalkoholpolyglykolethersulfosuccinate, Alkansulfonate, Fettsäureglutamate, Alkylsulfosuccinate, Fettsäuresarkoside; Fettsäuren, beispielsweise Palmitin-, Stearin- und Ölsäure; Seifen, beispielsweise Alkalisalze von Fettsäuren, Naphthensäuren und Harzsäuren, beispielsweise Abietinsäure, alkalilösliche Harze, beispielsweise kolophoniummodifizierte Maleinatharze und Kondensationsprodukte auf Basis von Cyanurchlorid, Taurin, N,N'-Diethylaminopropylamin und p-Phenylendiamin in Betracht. Besonders bevorzugt sind Harzseifen, d.h. Alkalisalze von Harzsäuren.
Als kationaktive Substanzen kommen beispielsweise quaternäre Ammoniumsalze, Fettaminoxalkylate, oxalkylierte Polyamine, Fettaminpolyglykolether, Fettamine, von Fettaminen oder Fettalkoholen abgeleitete Di- und Polyamine und deren Oxalkylate, von Fettsäuren abgeleitete Imidazoline, und Salze dieser kationenaktiven Substanzen, wie beispielsweise Acetate, in Betracht.
Als nichtionogene Substanzen kommen beispielsweise Aminoxide, Fettalkoholpolyglykolether, Fettsäurepolyglykolester, Betaine, wie Fettsäureamid-N-propyl-betaine, Phosphorsäureester von aliphatischen und aromatischen Alkoholen, Fettalkoholen oder Fettalkoholpolyglykolethern, Fettsäureamidethoxylate, Fettalkohol-alkylenoxid-Addukte und Alkylphenolpolyglykolether in Betracht.

Mit nichtpigmentären Dispergiermitteln sind Substanzen gemeint, die strukturell nicht durch chemische Modifikation von organischen Pigmente abgeleitet sind. Sie werden als Dispergiermittel entweder bereits bei der Herstellung von Pigmenten, oft aber auch bei der Einarbeitung der Pigmente in die zu färbenden Anwendungsmedien, beispielsweise bei der Herstellung von Lacken oder Druckfarben durch Dispergierung der Pigmente in den entsprechenden Bindemitteln, zugegeben. Es können polymere Substanzen, beispielsweise Polyolefine, Polyester, Polyether, Polyamide, Polyimine, Polyacrylate, Polyisocyanate, Block-Copolymere daraus, Copolymere aus den entsprechenden Monomeren oder Polymere einer Klasse, die mit wenigen Monomeren einer anderen Klasse modifiziert sind, sein. Diese polymeren Substanzen tragen polare Ankergruppen wie beispielsweise Hydroxy-, Amino-, Imino- und Ammoniumgruppen, Carbonsäure- und Carboxylatgruppen, Sulfonsäure- und Sulfonatgruppen oder Phosphonsäure- und Phosphonatgruppen, und können auch mit aromatischen, nicht pigmentären Substanzen modifiziert sein. Nichtpigmentäre Dispergiermittel können des weiteren auch chemisch mit funktionellen Gruppen modifizierte aromatische, nicht von organischen Pigmenten abgeleitete Substanzen sein. Derartige nichtpigmentäre Dispergiermittel sind dem Fachmann bekannt und zum Teil im Handel erhältlich (z.B. Solsperse^{®}, Avecia; Disperbyk^{®}, Byk, Efka^{®}, Efka). Es sollen im Folgenden stellvertretend einige Typen genannt werden, zum Einsatz können jedoch prinzipiell beliebige andere, beschriebene Substanzen kommen, beispielsweise Kondensationsprodukte aus Isocyanaten und Alkoholen, Di- oder Polyolen, Aminoalkoholen oder Di- oder Polyaminen, Polymere aus Hydroxycarbonsäuren, Copolymere aus Olefinenmonomeren oder Vinylmonomeren und ethylenisch ungesättigten Carbonsäureestern, urethanhaltige Polymere von ethylenisch ungesättigten Monomeren, urethanmodifizierte Polyester, Kondensationsprodukte auf Basis von Cyanurhalogeniden, Nitroxylverbindungen enthaltende Polymere, Polyesteramide, modifizierte Polyamide, modifizierte Acrylpolymere, Kammdispergiermittel aus Polyestern und Acrylpolymeren, Phosphorsäureester, von Triazin abgeleitete Polymere, modifizierte Polyether, oder von aromatischen, nichtpigmentären Substanzen abgeleitete Dispergiermittel. Dabei werden diese Grundstrukturen vielfach weiter modifiziert, beispielsweise durch chemische Umsetzung mit weiteren, funktionelle Gruppen tragenden Substanzen oder durch Salzbildung.

Mit pigmentären Dispergiermitteln sind Pigmentdispergatoren gemeint, die sich von einem organischen Pigment als Grundkörper ableiten und durch chemische Modifizierung dieses Grundkörpers hergestellt werden, beispielsweise saccharinhaltige Pigmentdispergatoren, piperidylhaltige Pigmentdispergatoren, von Naphthalen oder Perylen abgeleitete Pigmentdispergatoren, Pigmentdispergatoren mit funktionellen Gruppen, die über eine Methylengruppe mit dem Pigmentgrundkörper verknüpft sind, mit Polymeren chemisch modifizierte Pigmentgrundkörper, Sulfosäuregruppen haltige Pigmentdispergatoren, Sulfonamidgruppen haltige Pigmentdispergatoren, Ethergruppen haltige Pigmentdispergatoren, oder Carbonsäure-, Carbonsäureester- oder Carbonamidgruppen haltige Pigmentdispergatoren.

Zum Einstellen eines gewünschten pH-Wertes können Pufferlösungen zugeführt werden, vorzugsweise von organischen Säuren und deren Salzen, wie beispielsweise Ameisensäure/Formiat-Puffer, Essigsäure/Acetat-Puffer, Zitronensäure/Citrat-Puffer; oder von anorganischen Säuren und deren Salzen, wie beispielsweise Phosphorsäure/Phosphat-Puffer oder Kohlensäure/Hydrogencarbonat- bzw. Carbonat-Puffer.

Vorzugsweise wird das Azopigment direkt nach der Reaktion isoliert. Es ist aber auch möglich, eine Nachbehandlung (Finish) mit Wasser und/oder einem organischen Lösemittel durchzuführen, beispielsweise bei Temperaturen von 20 bis 250°C, gegebenenfalls auch unter Zusatz von Hilfsmitteln. Beispielsweise können zu diesem Zweck die feuchten oder getrockneten Pigmente in Wasser oder in organischen Lösemitteln, wie beispielsweise Pyridin, N-Methyl-2-pyrrolidon, Dimethylformamid, Dimethylacetamid, Dimethylsulfoxid, Alkoholen, Chlorbenzolen, Eisessig, Chinolin oder Glykolen, oder einer Mischung dieser Lösemittel einige Zeit, gegebenenfalls unter erhöhtem Druck und gegebenenfalls unter Zusatz von nichtionischen oder ionischen oberflächenaktiven Substanzen erhitzt werden.

Ein weiterer Gegenstand der vorliegenden Erfindung sind die Kupplungskomponenten der allgemeinen Formel (II), 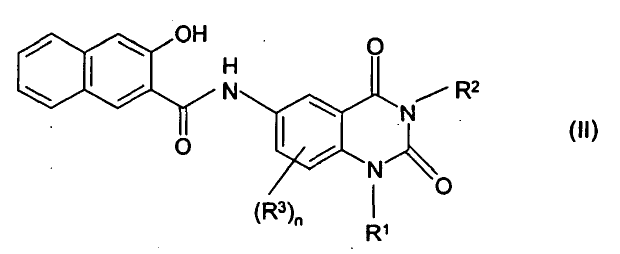 worin R¹, R² und R³ die vorstehend genannten Bedeutungen haben, sowie die entsprechenden tautomeren Formen und die jeweiligen Konfigurationsisomere jeder tautomeren Form.

Die Verbindungen der Formel (II) können hergestellt werden, indem 2-Hydroxy-3-naphthoesäure oder 2-Hydroxy-3-naphthoesäurechlorid mit einem Amin der allgemeinen Formel (IV) kondensiert wird, vorzugsweise bei Temperaturen zwischen 0 und 80°C und in Gegenwart von PCl₃ und einer Base, wie z.B. Pyridin.

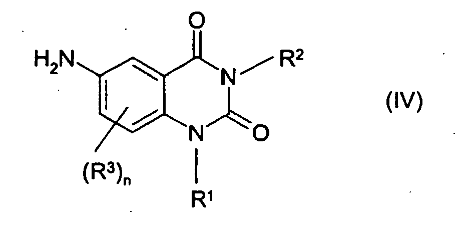

Die erfindungsgemäßen Verbindungen der Formel (I) eignen sich besonders zum Pigmentieren von Lacken, Kunststoffen, Druckfarben, elektrophotographischen Tonern und Entwicklern, Pulverlacken, Tinten, vorzugsweise Ink-Jet-Tinten, wässrigen und nichtwässrigen Pigmentpräparationen, Farbfiltern und zum Einfärben von Saatgut.
Beispielsweise können natürliche oder synthetische hochmolekulare organische Materialien, wie beispielsweise Celluloseether und -ester, wie Ethylcellulose, Nitrocellulose, Celluloseacetat oder Cellulosebutyrat, natürliche Harze oder Kunstharze, wie Polymerisationsharze oder Kondensationsharze, beispielsweise Aminoplaste, insbesondere Harnstoff- und Melamin-Formaldehydharze, Alkydharze, Acrylharze, Phenoplaste, Polycarbonate, Polyolefine, wie Polystyrol, Polyvinylchlorid, Polyethylen, Polypropylen, Polyacrylnitril, Polyacrylsäureester, Polyamide, Polyurethane oder Polyester, Gummi, Latices, Casein; Silikone und Silikonharze, einzeln oder in Mischungen, pigmentiert werden.
Dabei spielt es keine Rolle, ob die erwähnten hochmolekularen organischen Verbindungen als plastische Massen, Gießharze, Pasten, Schmelzen oder in Form von Spinnlösungen, Lacken, Lasuren, Schäumen, Tuschen, Tinten, Beizen, Anstrichstoffen, Dispersionsfarben oder Druckfarben vorliegen. Je nach Verwendungszweck erweist es sich als vorteilhaft, die erfindungsgemäßen Azofarbmittel als Blends oder in Form von Präparationen oder Dispersionen zu benutzen. Bezogen auf das zu färbende, hochmolekulare organische Material setzt man die erfindungsgemäß hergestellten Azofarbmittel in einer Menge von vorzugsweise 0,05 bis 30 Gew.-%, vorzugsweise 0,1 bis 15 Gew.-% ein.
Mit den erfindungsgemäßen Azopigmenten können beispielsweise die technisch gängigen Einbrennlacke aus der Klasse der Alkyd-Melamin-Harzlacke, Acryl-Melamin-Harzlacke, Polyesterlacke, Highsolidacrylharzlacke, wässrige Lacke auf Polyurethanbasis sowie Zweikomponentenlacke auf Basis polyisocyanatvernetzbarer Acrylharze und insbesondere Automobil-Metallic-Lacke pigmentiert werden.
Die erfindungsgemäßen Azofarbmittel sind auch geeignet als Farbmittel in elektrophotographischen Tonern und Entwicklern, wie z.B. Ein- oder Zweikomponentenpulvertonern (auch Ein- oder Zweikomponenten-Entwickler genannt), Magnettoner, Flüssigtoner, Polymerisationstoner sowie Spezialtoner. Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.
Des weiteren sind die erfindungsgemäßen Azofarbmittel geeignet als Farbmittel in Pulver und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen. Als Pulverlackharze werden typischerweise Epoxidharze, carbonyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Außerdem sind die erfindungsgemäßen Azofarbmittel als Farbmittel in Ink-Jet Tinten auf wässriger und nichtwässriger Basis sowie in solchen Tinten, die nach dem Hot-melt-Verfahren arbeiten, geeignet.

Darüber hinaus sind die erfindungsgemäßen Azofarbmittel auch als Farbmittel für Farbfilter, sowohl für die subtraktive als auch für die additive Farberzeugung, geeignet.

Für die genannten Anwendungen können die Verbindungen der Formel (I) als Pulver, Presskuchen oder Flush-Pasten eingesetzt werden.

Die erfindungsgemäßen Verbindungen der Formel (I) zeichnen sich durch brillante Farbtöne, hohe Licht-, Lösemittel- und Überlackierechtheiten, Temperaturstabilität, hohe Farbstärken, gute Dispergierbarkeit im Anwendungsmedium sowie gute rheologische Eigenschaften aus.

Zur Beurteilung der Eigenschaften der nach der Erfindung hergestellten Pigmente auf dem Lacksektor wurden aus der Vielzahl der bekannten Lacke ein aromatenhaltiger Alkydmelaminlack (AM) auf Basis eines mittelöligen, nichttrocknenden Alkydharzes ausgewählt.

In den folgenden Beispielen bedeuten Teile Gewichtsteile und Prozente Gewichtsprozente.

Herstellung von 1-Methyl-6-(2'-hydroxy-3'-naphthoylamino)chinazolin-2,4-dion:
50 Teile 2-Hydroxy-3-naphthoesäure und 51.6 Teile 6-Amino-1-methylchinazolin-2,4-dion werden in 200 Teilen Pyridin suspendiert und innerhalb von 30 min bei 30 bis 50°C 20,4 Teile Phosphortrichlorid zugetropft. Anschließend wird 1 h bei 100°C gerührt, mit 300 Teilen Wasser versetzt und das Pyridin mit Wasserdampf ausgetrieben. Nach dem Abkühlen auf Raumtemperatur wird abgesaugt, mit Wasser gewaschen und getrocknet. Man erhält 67 Teile eines ockerfarbenen Feststoffes.

### Beispiel 1

9,7 Teile Anthranilsäurebutylester werden in 50 Teilen Wasser und 17,3 Teilen 31 %iger Salzsäure mit 8,8 Teilen 40 %iger Natriumnitrit-Lösung bei 5 bis 10°C diazotiert, mit 200 Teilen Wasser verdünnt, geklärt und der Nitritüberschuss beseitigt. Dann wird die Diazoniumsalzlösung mit 10 Teilen einer 10%igen Lösung von ^{®}Genapol T250 in Wasser und 200 Teilen einer 4 molaren Natriumacetatlösung versetzt. 18,97 Teile 1-Methyl-6-(2'-hydroxy-3'-naphthoylamino)chinazolin-2,4-dion werden in 600 Teilen Wasser und 20,4 Teilen 33 %iger Natronlauge gelöst. Die auf diese Weise bereitete Kupplerlösung wird innerhalb von 25 min zur Diazoniumsalzlösung dosiert. Danach wird bei einem pH-Wert von 6,2 bei 30°C gerührt, bis kein Diazoniumsalz mehr nachweisbar ist und weitere 30 min bei 94°C gerührt, abfiltriert und mit Wasser gewaschen. Der feuchte Presskuchen wird in
855 Teilen Dimethylformamid suspendiert und zur Nachbehandlung bei 100°C gerührt. Anschließend wird abfiltriert, gewaschen, getrocknet und gemahlen. Man erhält 21,0 Teile eines roten Pigmentes.

### Beispiel 2

6,8 Teile p-Aminobenzamid werden in 50 Teilen Wasser und 17,3 Teilen 31 %iger Salzsäure gelöst und mit 8,8 Teilen 40 %iger Natriumnitrit-Lösung bei 0 bis 5°C diazotiert, mit 200 Teilen Wasser verdünnt und der Nitritüberschuss beseitigt. Dann wird die Diazoniumsalzlösung mit 10 Teilen einer 10 %igen Lösung von ^{®}Genapol T250 in Wasser und 200 Teilen einer 4 molaren Natriumacetatlösung versetzt. 18,97 Teile 1-Methyl-6-(2'-hydroxy-3'-naphthoylamino)chinazolin-2,4-dion werden in 600 Teilen Wasser und 20,4 Teilen 33 %iger Natronlauge gelöst. Die auf diese Weise bereitete Kupplerlösung wird innerhalb 25 min zur Diazoniumsalzlösung dosiert. Danach wird bei einem pH-Wert von 5,5 bis 6,0 nachgerührt, bis kein Diazoniumsalz mehr nachweisbar ist und weitere 30 min bei 94°C gerührt, abfiltriert, mit Wasser gewaschen und getrocknet. Das getrocknete Rohpigment wird gemahlen und zur Nachbehandlung in 500 Teilen N-Methylpyrrolidon suspendiert und für 25 min bei 106°C gerührt. Anschließend wird abfiltriert, gewaschen, getrocknet und gemahlen. Man erhält 17,4 Teile eines roten Pigmentes.

### Beispiel 3

11,5 Teile 4-Amino-5-methoxy-2-methylbenzol-N-methylsulfonamid werden in 50 Teilen Wasser und 17,3 Teilen 31 %iger Salzsäure gelöst und mit 8,8 Teilen 40 %iger Natriumnitrit-Lösung bei 0 bis 5°C diazotiert, mit 200 Teilen Wasser verdünnt und der Nitritüberschuss beseitigt. Dann wird die Diazoniumsalzlösung mit 10 Teilen einer 10 %igen Lösung von ^{®}Genapol T250 in Wasser und 200 Teilen einer 4 molaren Natriumacetatlösung versetzt. 18,97 Teile 1-Methyl-6-(2'-hydroxy-3'-naphthoylamino)chinazolin-2,4-dion werden in 600 Teilen Wasser und 20,4 Teilen
33 %iger Natronlauge gelöst. Die auf diese Weise bereitete Kupplerlösung wird innerhalb 25 min zur Diazoniumsalzlösung dosiert. Danach wird bei einem pH-Wert von 5,5 bis 6,0 nachgerührt, bis kein Diazoniumsalz mehr nachweisbar ist und weitere 30 min bei 94°C gerührt, abfiltriert und mit Wasser gewaschen. Der feuchte Presskuchen wird in 700 Teilen Essigsäure suspendiert und zur Nachbehandlung bei 100°C gerührt. Anschließend wird abfiltriert, gewaschen, getrocknet und gemahlen. Man erhält 27,6 Teile eines roten Pigmentes.

Man verfährt in Analogie zu Beispiel 1 und setzt als Kupplungskomponente das oben hergestellte 1-Methyl-6-(2'-hydroxy-3'-naphthoylamino)chinazolin-2,4-dion sowie die in Tabelle 1 aufgeführten Diazokomponenten ein:

**Tabelle 1:**

| Beispiel-Nr. | Diazokomponente | Farbton |
|---|---|---|
| 1 | 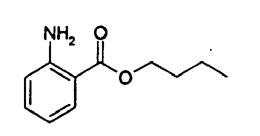 | rot/gelbstichig |
| 2 | 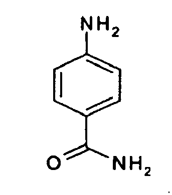 | rot/neutral |
| 3 | 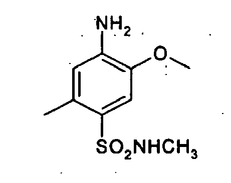 | rot/neutral |
| 4 | 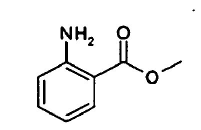 | rot/neutral |
| 5 | 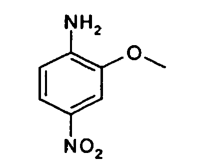 | rot/blaustichig |
| 6 | 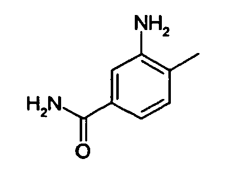 | rot/gelbstichig |
| | | |
| 7 | 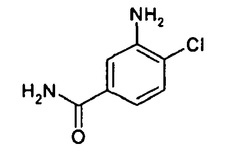 | rot/neutral |
| 8 | 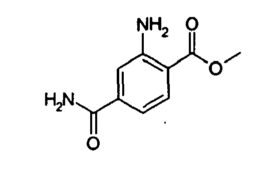 | rot/gelbstichig |
| 9 | 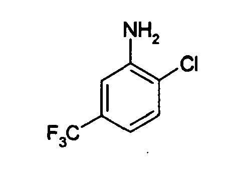 | rot/neutral |
| 10 | 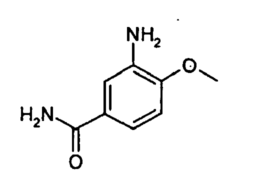 | rot/neutral |

### Vergleichsbeispiel 1

Bei der Herstellung des Pigmentes wird verfahren wie unter Beispiel 2 beschrieben, nur dass anstatt des 1-Methyl-6-(2'-hydroxy-3'-naphthoylamino)-chinazolin-2,4-dions 6-(2'-Hydroxy-3'-naphthoylamino)chinazolin-2,4-dion eingesetzt wird. Man erhält 28,1 g eines roten Pigmentes.

### Vergleichsbeispiel 2:

Bei der Herstellung des Pigmentes wird verfahren wie unter Beispiel 3 beschrieben, nur dass anstatt des 1-Methyl-6-(2'-hydroxy-3'-naphthoylamino) chinazolin-2,4-dions 6-(2'-Hydroxy-3'-naphthoylamino)chinazolin-2,4-dion eingesetzt wird. Man erhält 28,5 g eines roten Pigmentes.

Zur Beurteilung der anwendungstechnischen Eigenschaften der erfindungsgemäßen Pigmente wurde ein Alkyd-Melamin-Lack-System (AM) ausgewählt. Die erfindungsgemäßen Pigmente zeichnen sich gegenüber den Vergleichsbeispielen durch deutlich höhere Farbstärken, reinere Farbtöne sowie deutlich höhere Deckkraft aus.

| Beispiel 2 im Vergleich zu Vergleichsbeispiel 1: | |
|---|---|
| Farbstärke: | 195 % |
| Reinheitsunterscheid (Chroma): | dC = 3,49 |

| Beispiel 3 im Vergleich zu Vergleichsbeispiel 2: | |
|---|---|
| Farbstärke: | 115 % |
| Reinheitsunterscheid (Chroma): | dC = 5,52 |

## Patentansprüche

1. Azopigment der allgemeinen Formel (I), 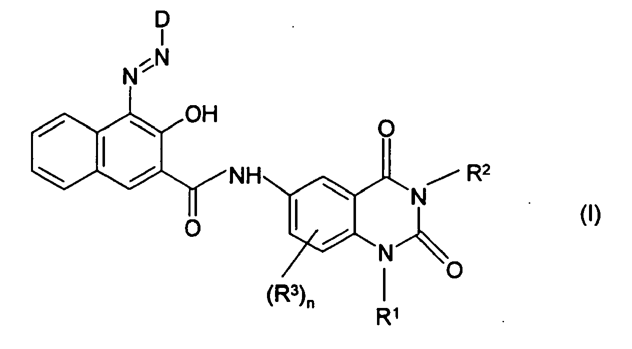 worin
D den Rest einer Diazokomponente darstellt,
R¹ und R² gleich oder verschieden sind und Wasserstoff, einen gegebenenfalls durch Hydroxy, Amino, Halogen oder Methoxy substituierten C₁-C₄-Alkylrest, oder einen gegebenenfalls mit 1, 2 oder 3 Substituenten aus der Gruppe C₁-C₄-Alkyl, C₁-C₄-Alkoxy, F, Cl, Br, CF₃, NO₂, OH, CONH₂, CONH(C₁-C₄-alkyl), CON(C₁-C₄-alkyl)₂, COO(C₁-C₄-alkyl), SO₂NH₂, SO₂NH(C₁-C₄-alkyl) und SO₂N(C₁-C₄-alkyl)₂ substituierten (C₆-C₁₀)-Arylrest bedeuten,
mit der Maßgabe, dass R¹ und R² nicht gleichzeitig Wasserstoff sind;
R³ Wasserstoff, C₁-C₄-Alkyl, Halogen, Trifluormethyl, C₁-C₄-Alkoxy, NO₂, oder CN bedeutet, und n die Zahl 1, 2 oder 3 ist.

2. Azopigment gemäß Anspruch 1, **dadurch gekennzeichnet, dass** R¹ und R² Wasserstoff, Methyl, Ethyl oder Phenyl bedeuten.

3. Azopigment gemäß Anspruch 1 oder 2, **dadurch gekennzeichnet, dass** R¹ Methyl oder Ethyl, und R² Wasserstoff bedeuten.

4. Azopigment nach einem oder mehreren der Ansprüche 1 bis 3, **dadurch gekennzeichnet, dass** R³ Wasserstoff, Methyl, Ethyl, Methoxy, Ethoxy, F, Cl, CF₃ oder Nitro bedeutet.

5. Azopigment nach einem oder mehreren der Ansprüche 1 bis 4, **dadurch gekennzeichnet, dass** D einen (C₆-C₁₂)-Arylrest, der mit 1, 2 oder 3 Substituenten aus der Gruppe C₁-C₄-Alkyl, C₁-C₄-Alkoxy, Phenyl, Chlorphenyl, Dichlorphenyl, F, Cl, Br, CF₃, NO₂, NH₂, COOH, OH, CONH₂, CONH(C₁-C₄-alkyl), CON(C₁-C₄-alkyl)₂, COO(C₁-C₄-alkyl), SO₂NH₂, SO₂NH(C₁-C₄-alkyl) und SO₂N(C₁-C₄-alkyl)₂ substituiert ist, oder ein (C₆-C₁₀)-Arylrest, an den ein fünf- oder sechsgliedriger heterocyclischer Ring mit 1, 2 oder 3 Heteroatomen aus der Gruppe N, O und S annelliert ist, bedeutet.

6. Verfahren zur Herstellung eines Azopigments nach einem oder mehreren der Ansprüche 1 bis 5, **dadurch gekennzeichnet, dass** man ein Amin der Formel D-NH₂ diazotiert und mit einer Verbindung der Formel (II) kuppelt

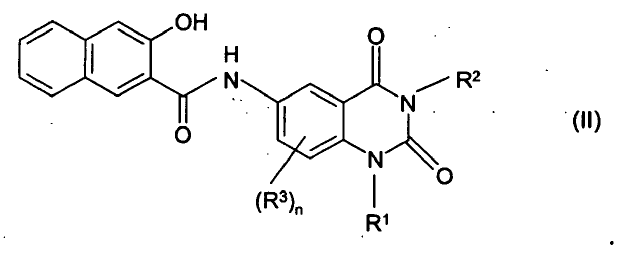

7. Verbindung der Formel (II) 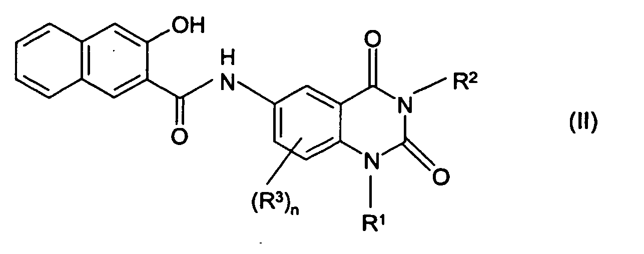 worin
R¹ und R² gleich oder verschieden sind und Wasserstoff, einen gegebenenfalls durch Hydroxy, Amino, Halogen oder Methoxy substituierten C₁-C₄-Alkylrest, oder einen gegebenenfalls mit 1, 2 oder 3 Substituenten aus der Gruppe C₁-C₄-Alkyl, C₁-C₄-Alkoxy, F, Cl, Br, CF₃, NO₂, OH, CONH₂, CONH(C₁-C₄-alkyl), CON(C₁-C₄-alkyl)₂, COO(C₁-C₄-alkyl), SO₂NH₂, SO₂NH(C₁-C₄-alkyl) und SO₂N(C₁-C₄-alkyl)₂ substituierten (C₆-C₁₀)-Arylrest bedeuten,
mit der Maßgabe, dass R¹ und R² nicht gleichzeitig Wasserstoff sind;
R³ Wasserstoff, C₁-C₄-Alkyl, Halogen, Trifluormethyl, C₁-C₄-Alkoxy, NO₂, oder CN bedeutet, und
n die Zahl 1, 2 oder 3 ist.

8. Verwendung der Azopigmente gemäß Anspruch 1 bis 5 zum Pigmentieren von Lacken, Kunststoffen, Druckfarben, elektrophotographischen Tonern und Entwicklern, Pulverlacken, Tinten, vorzugsweise Ink-Jet-Tinten, wässrigen und nichtwässrigen Pigmentpräparationen, Farbfiltern und zum Einfärben von Saatgut.

## Claims

1. An azo pigment of the formula (I), 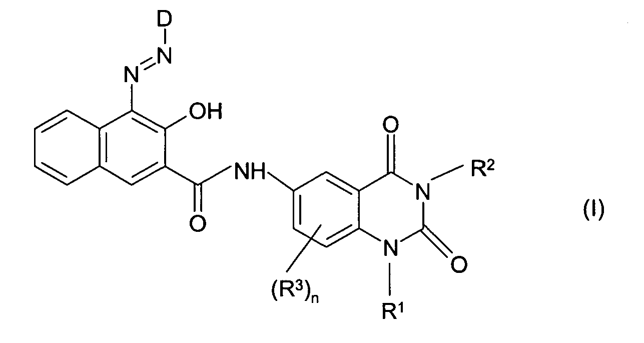 in which
D is the radical of a diazo component,
R¹ and R² are identical or different and are hydrogen, an optionally hydroxy-, amino-, halo- or methoxy-substituted C₁-C₄-alkyl radical, or a (C₆-C₁₀)-aryl radical which is unsubstituted or substituted by 1, 2 or 3 substituents from the group consisting of C₁-C₄-alkyl, C₁-C₄-alkoxy, F, Cl, Br, CF₃, NO₂, OH, CONH₂, CONH(C₁-C₄-alkyl), CON (C₁-C₄-alkyl)₂, COO(C₁-C₄-alkyl), SO₂NH₂, SO₂NH(C₁-C₄-alkyl) and SO₂N(C₁-C₄-alkyl)₂,
with the proviso that R¹ and R² are not simultaneously hydrogen;
R³ is hydrogen, C₁-C₄-alkyl, halo, trifluoromethyl, C₁-C₄-alkoxy, NO₂, or CN, and
n is 1, 2 or 3.

2. An azo pigment as claimed in claim 1, wherein R¹ and R² are hydrogen, methyl, ethyl or phenyl.

3. An azo pigment as claimed in claim 1 or 2, wherein R¹ is methyl or ethyl and R² is hydrogen.

4. An azo pigment as claimed in one or more of claims 1 to 3, wherein R³ is hydrogen, methyl, ethyl, methoxy, ethoxy, F, Cl, CF₃ or nitro.

5. An azo pigment as claimed in one or more of claims 1 to 4, wherein D is a (C₆-C₁₂)-aryl radical substituted by 1, 2 or 3 substituents from the group consisting of C₁-C₄-alkyl, C₁-C₄-alkoxy, phenyl, chlorophenyl, dichlorophenyl, F, Cl, Br, CF₃, NO₂, NH₂, COOH, OH, CONH₂, CONH(C₁-C₄-alkyl), CON(C₁-C₄-alkyl)₂, COO(C₁-C₄-alkyl), SO₂NH₂, SO₂NH(C₁-C₄-alkyl) and SO₂N(C₁-C₄-alkyl)₂ or is a (C₆-C₁₀)-aryl radical, to which a five- or six-membered heterocyclic ring having 1, 2 or 3 heteroatoms from the group consisting of N, O und S is fused.

6. A process for preparing an azo pigment as claimed in one or more of claims 1 to 5, which comprises diazotizing an amine of formula D-NH₂ and coupling the product with a compound of the formula (II)

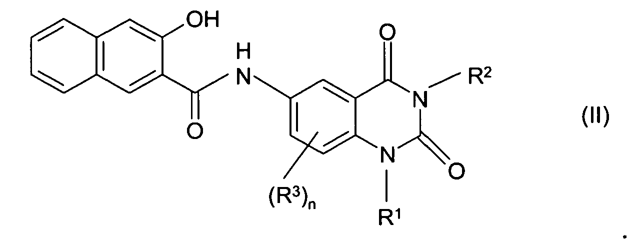

7. A compound of the formula (II) 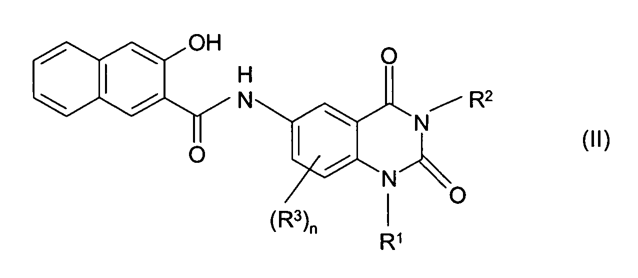 in which
R¹ and R² are identical or different and are hydrogen, an optionally hydroxy-, amino-, halo- or methoxy-substituted C₁-C₄-alkyl radical, or a (C₆-C₁₀)-aryl radical which is unsubstituted or substituted by 1, 2 or 3 substituents from the group consisting of C₁-C₄-alkyl, C₁-C₄-alkoxy, F, Cl, Br, CF₃, NO₂, OH, CONH₂, CONH(C₁-C₄-alkyl), CON(C₁-C₄-alkyl)₂, COO(C₁-C₄-alkyl), SO₂NH₂, SO₂NH(C₁-C₄-alkyl) and SO₂N(C₁-C₄-alkyl)₂,
with the proviso that R¹ and R² are not simultaneously hydrogen;
R³ is hydrogen, C₁-C₄-alkyl, halo, trifluoromethyl, C₁-C₄-alkoxy, NO₂, or CN, and
n is 1, 2 or 3.

8. The use of an azo pigment as claimed in claim 1 to 5 for pigmenting varnishes, plastics, printing inks, electrophotographic toners and developers, powder coating materials, graphics inks, preferably ink-jet inks, aqueous and nonaqueous pigment preparations, and color filters and for coloring seed.

## Revendications

1. Pigment azoïque de formule générale (I), 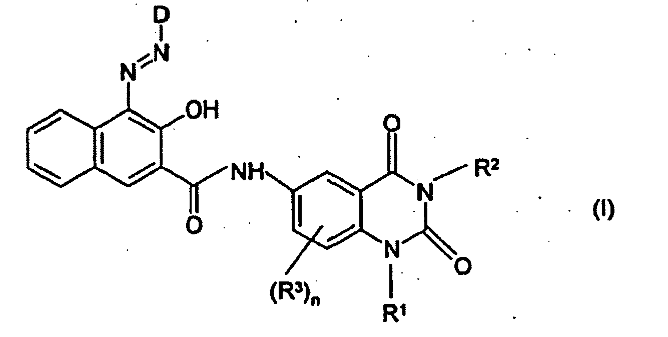 dans laquelle
D représente le radical d'un composant diazotable,
R¹ et R² sont identiques ou différents et représentent un atome d'hydrogène, un radical alkyle en C₁-C₄ éventuellement substitué par hydroxy, amino, halogène ou méthoxy, ou un radical aryle en C₆-C₁₀ portant éventuellement 1, 2 ou 3 substituants choisis dans l'ensemble constitué par F, Cl, Br et des groupes alkyle en C₁-C₄, alcoxy en C₁-C₄, CF₃, NO₂, OH, CONH₂, CONH [alkyle (C₁-C₄)], CON [alkyle (C₁-C₄)]₂, COO[alkyle(C₁-C₄)], SO₂NH₂, SO₂NH [alkyle (C₁-C₄)] et SO₂N [alkyle(C₁-C₄)]₂,
étant entendu que R¹ et R² ne représentent pas simultanément des atomes d'hydrogène ;
R³ représente un atome d'hydrogène ou d'halogène ou un groupe alkyle en C₁-C₄, trifluorométhyle, alcoxy en C₁-C₄, NO₂ ou CN, et
n est le nombre 1, 2 ou 3.

2. Pigment azoïque selon la revendication 1, **caractérisé en ce que** R¹ et R² représentent un atome d'hydrogène ou le groupe méthyle, éthyle ou phényle.

3. Pigment azoïque selon la revendication 1 ou 2, **caractérisé en ce que** R¹ représente le groupe méthyle ou éthyle, et R² représente un atome d'hydrogène.

4. Pigment azoïque selon une ou plusieurs des revendications 1 à 3, **caractérisé en ce que** R³ représente un atome d'hydrogène, le groupe méthyle, éthyle, méthoxy, éthoxy,F, Cl, CF₃ ou nitro.

5. Pigment azoïque selon une ou plusieurs des revendications 1 à 4, **caractérisé en ce que** D représente un radical aryle en C₆-C₁₂ qui porte 1, 2 ou 3 substituants choisis dans l'ensemble constitué par les groupes alkyle en C₁-C₄, alcoxy en C₁-C₄, phényle, chlorophényle, dichlorophényle, F, Cl, Br, CF₃, NO₂, NH₂, COOH, OH, CONH₂, CONH [alkyle (C₁-C₄)], CON[alkyle(C₁-C₄)]₂, COO[alkyle(C₁-C₄)], SO₂NH₂, SO₂NH [alkyle(C₁-C₄)] et SO₂N[alkyle(C₁-C₄)]₂, ou un radical aryle en C₆-C₁₀ auquel est soudé un cycle hétérocyclique à cinq ou six chaînons comportant 1, 2 ou 3 hétéroatomes choisi parmi N, O et S.

6. Procédé pour la préparation d'un pigment azoïque selon une ou plusieurs des revendications 1 à 5, **caractérisé en ce qu'**on soumet à une diazotation une amine de formule D-NH₂ et on la fait copuler avec un composé de formule (II)

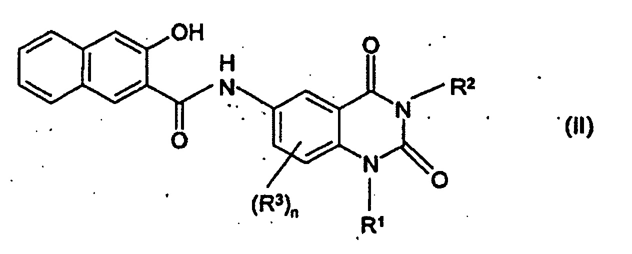

7. Composé de formule (II) 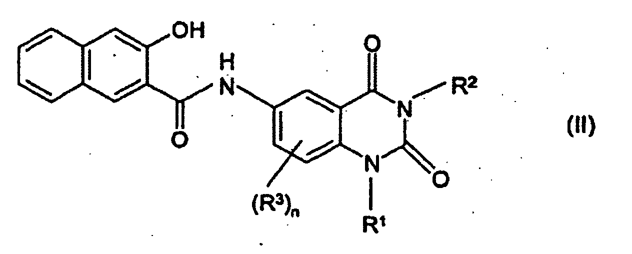 dans laquelle
R¹ et R² sont identiques ou différents et représentent un atome d'hydrogène, un radical alkyle en C₁-C₄ éventuellement substitué par hydroxy, amino, halogène ou méthoxy, ou un radical aryle en C₆-C₁₀ portant éventuellement 1, 2 ou 3 substituants choisis dans l'ensemble constitué par F, Cl, Br et des groupes alkyle en C₁-C₄, alcoxy en C₁-C₄, CF₃, NO₂, OH, CONH₂, CONH [alkyle(C₁-C₄)], CON [alkyle(C₁-C₄)]₂, COO[alkyle(C₁-C₄)], SO₂NH₂, SO₂NH[alkyle(C₁-C₄)] et SO₂N [alkyle(C₁-C₄)]₂,
étant entendu que R¹ et R² ne représentent pas en même temps des atomes d'hydrogène ;
R³ représente un atome d'hydrogène ou d'halogène ou un groupe alkyle en C₁-C₄, trifluorométhyle, alcoxy en C₁-C₄, NO₂ ou CN, et
n est le nombre 1, 2 ou 3.

8. Utilisation des pigments azoïques selon l'une quelconque des revendications 1 à 5, pour la pigmentation de peintures, de matières plastiques, d'encres d'imprimerie, de toners et développeurs électrophotographiques, de peintures en poudre, d'encres, de préférence d'encres pour impression à jet d'encre, de préparations de pigments aqueuses ou non aqueuses, de filtres colorés et pour la coloration de semences.
